(19) Europäisches Patentamt
European Patent Office
Office européen des brevets

(11) Publication number: **0 408 069 A2**

# (12) EUROPEAN PATENT APPLICATION

(21) Application number: **90113476.7**

(51) Int. Cl.5: **A61K 9/00**, A61K 47/36

(22) Date of filing: **13.07.90**

(30) Priority: **14.07.89 US 379949**

(43) Date of publication of application:
**16.01.91 Bulletin 91/03**

(84) Designated Contracting States:
**AT BE CH DE DK ES FR GB GR IT LI LU NL SE**

(71) Applicant: **UNION CARBIDE CHEMICALS AND PLASTICS COMPANY, INC.**
**39 Old Ridgebury Road**
**Danbury Connecticut 06817-0001(US)**

(72) Inventor: **Brode, George Lewis II**
**653 Carlene Drive**
**Bridgewater N.J. 08807(US)**
Inventor: **Salensky, George Antony**
**10 Scabbletown Road**
**White House Station N.J. 08889(US)**
Inventor: **Harris, William Charles, Jr.**
**62 Rohill Road**
**Neshanic Station N.J. 08853(US)**

(74) Representative: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**D-8000 München 40(DE)**

## (54) Emulsions comprising aminopolysaccharides.

(57) A biocompatible, substantive, film-forming delivery system for delivery of pharmaceutical or therapeutic actives to a desired topical site of a subject, said system comprising an emulsion comprising

A) at least one pharmaceutical or therapeutic active;

B) an aqueous fraction comprising at least one aminopolysaccharide derivative selected from the group consisting of chitosonium polymers and covalent chitosan derivatives; and

C) a non-aqueous fraction which is not soluble in the aqueous fraction,

and wherein said system, after delivery to said site, provides a substantive, gas permeable film from which the actives are available for treatment of said subject at said site.

EP 0 408 069 A2

# EMULSIONS COMPRISING AMINOPOLYSACCHARIDES

## FIELD OF THE INVENTION

This invention relates, in general, to novel delivery systems useful for the topical delivery of emulsions which may contain pharmaceutical or therapeutic actives. In one aspect, this invention relates to delivery systems containing certain aminopolysaccharides and derivatives thereof which are effective systems for the delivery of emulsions which may contain a variety of pharmaceutical and therapeutic actives. In further aspects, this invention is directed to the preparation of such systems and to a method for the topical administration of a pharmaceutical or therapeutical active to a subject using said system.

## BACKGROUND OF THE INVENTION

Traditionally, pharmaceutical and therapeutic actives can be administered to the body by a number of routes, including ingestion, injection, inhalation, and topical application. Absorption of an active by ingestion, injection, or inhalation generally gives systemic distribution of the active throughout the body. Systemic distribution of the active may be unsatisfactory for three reasons. First, these modes of administration produce non-specific distribution. The active is distributed through the entire body and not localized. Second, there may be undesirable effects such as toxic or irritating reactions on non-target organs or regions. Finally, to achieve the desired effect at the target organ or region, a higher dosage than might otherwise be desired must be administered to compensate for systemic dilution of the active.

In contrast to systemic delivery, topical delivery is application of an active in a manner so that it acts primarily at the site of application. This type of application is used typically for dermatological disorders. The above-described deficiencies of systemic delivery are not encountered when an active is applied topically. Rather, topical application affords the opportunity to minimize the dosage and confine the active to the region of the body to which it is applied. Thus, systemic distribution of the active throughout the body is obviated. For example, ingestion of acetone could cause fetal problems which dermal delivery should avoid. Typical sites of topical delivery include application to the dermal, opththalmic, and mucous membranes and tissues, such as the hair, skin, eyes, ears, mouth, nose, throat, rectum, vagina, and urethra.

However, despite these advantages of topical delivery, most current topical delivery formulations are inefficient and therefore have limited utility. There are four reasons for this inefficiency of current topical delivery technology. First, skin and mucous membranes possess good barrier properties and the permeability of most actives through these barriers generally is poor. Second, actives applied topically are subject to migration and loss due to perspiration, natural tissue lavation, and mechanical action. Third, because most pharmaceutical or therapeutic actives are relatively simple, low molecular weight compounds or mixtures, these actives are not applied alone, but in combination with a variety of additives to deliver the active to the application site and control the dosage. Fourth, the choice of a proper delivery system can minimize undesirable crystallization of the active, and hence optimize its availability in its active form. Most known topical delivery systems are petrolatum-based cremes and ointments. These unctuous formulations are unsatisfactory because they are at best uncomfortable and messy when applied.

A topical delivery system cannot be considered fully satisfactory if it is deficient with regard to any of the above-described criteria. For example, a delivery system which does not ensure that the active efficiently penetrates the application site is not satisfactory because it requires that an excess of active be incorporated into the delivery system to ensure delivery of an effective quantity. The remaining active, i.e., that which does not penetrate the application site, is wasted. Similarly, active which is allowed to migrate from the application site, or to crystallize before it penetrates the site, is wasted. Further, a delivery system which satisfies each criterion will be adjudged a failure by a consumer who is dissatisfied because the delivery system leaves an unpleasant residue. For example, an unctuous residue, which is unpleasant to the touch and messy, may cause a consumer not to utilize the treatment. Thus, such delivery systems are unsatisfactory.

## SUMMARY OF THE INVENTION

The present invention is directed to novel delivery systems comprising emulsions of an aqueous solution of an aminopolysaccharide derivative and a non-aqueous phase. The invention also relates to a

method for preparing the delivery systems, to a method for their application to a subject, and to a method of testing their substantivity.

The delivery system of the present invention is a biocompatible, substantive, film-forming system for the delivery of pharmaceutical or therapeutic actives to a desired topical site of a subject or host. The system comprises:

A) at least one pharmaceutical or therapeutic active;

B) an aqueous fraction comprising at least one aminopolysaccharide derivative selected from the group consisting of chitosonium polymers and covalent chitosan derivatives; and

C) a non-aqueous fraction which is not soluble in the aqueous fraction.

The system efficiently delivers the actives to the user at the application site and provides at the site a non-irritating, essentially imperceptible, substantive, gas permeable film over the application site.

## DETAILED DESCRIPTION OF THE INVENTION

This invention is based on the discovery that a delivery system for pharmaceutical or therapeutical actives comprising:

A) at least one pharmaceutical or therapeutic active;

B) an aqueous fraction comprising at least one aminopolysaccharide derivative selected from the group consisting of chitosonium polymers and covalent chitosan derivatives; and

C) a non-aqueous fraction which is not soluble in the aqueous fraction;

is, unexpectedly, substantive to proteinaceous substrates, such as hair, skin, and mucosa.

The aqueous fraction of the delivery system of this invention encompasses divers aminopolysaccharides, particularly chitosan derivatives. The non-aqueous fraction encompasses at least one component which is not water-soluble so that an emulsion is formed. Each fraction can contain other components soluble therein. Together, the two fractions possess a variety of useful characteristics which make these materials superior for the delivery of pharmaceutical and therapeutic actives.

As used throughout the specification and claims, the phrase "pharmaceutical active" is considered to be a drug, i.e., a substance which, when applied to or introduced into the body, alters body functions in some way. The phrase "therapeutic active" is broader in scope and includes any substance which is capable of altering either body function or cosmetic appearance, but which is not traditionally or technically considered a drug. For example, mineral oil does alter the skin in at least a cosmetic manner or in some cases may be therapeutic. Therefore, mineral oil is considered to be a "therapeutic active" for purposes of the present invention.

There are several features which make the delivery systems of the present invention superior delivery vehicles. In the first instance, surprisingly, the delivery systems of this invention are substantive with hair, skin, and mucous membrane. Throughout the specification and claims, the term "substantive" means that there exists a cohesive interaction between the aminopolysaccharide and a proteinaceous substrate, i.e., the hair, skin, or mucosa, to which the delivery system is applied. In the delivery systems of the present invention, substantivity typically is obtained by ensuring that the aminopolysaccharide is cationically charged. The cationic charge is imparted by protonation or quaternization of the aminopolysaccharide. Incorporation of appropriate hydrophobic groups or combinations thereof may be used. Thus, the delivery systems of the present invention exhibit a cohesive interaction with the proteins of hair, skin, and mucosa.

Aminopolysaccharides, particularly chitosan derivatives, are good film-formers, i.e., a polymeric film is readily formed when an aqueous solution of a water-soluble aminopolysaccharide is applied topically. Upon topical application of the delivery system of this invention, a polymeric film forms and serves as a reservoir from which the active is continuously delivered. The film also serves to protect the application site from insult or injury.

Cationically-charged chitosan derivatives exhibit substantive properties to keratin and other protein constituents of hair, skin, and mucosa. Thus, upon application of a cationic chitosan derivative to these tissues, the resulting film is bound to the substrate. This close relationship minimizes loss or migration of the film and the active. Any two-phase form of the delivery system, such as creme, ointment spray (aerosol, for example) comprising the two fractions conveniently may be utilized to form the subject delivery system.

Application of an active- and aminopolysaccharide-containing delivery system which forms a film provides uniform distribution of the active on the tissue and prevents migration or loss of the active from the site of application. The reservoir of active in the film helps to control the dosage at a constant level, thus controlling the rate of release. Also, chitosan derivatives which are free of naturally-associated proteins, heavy metals, and the like, are biocompatible and non-irritating to living tissue. These derivatives also do

not elicit an inflammatory, allergic, or pyrogenic response in humans. In addition, the films these chitosan derivatives form on skin and mucosa are essentially imperceptible to the patient and cosmetically comfortable to wear. Further, the films are gas-permeable.

The chitosan derivatives are also good humectants. Moisturization of the skin and mucous membranes enhances the absorption and permeation of many pharmaceutical and therapeutic actives into those tissues. When these chitosan derivatives are applied to skin or mucous membranes, their humectant properties therefore enhance the absorption of the actives into these tissues.

As indicated above, there are two types of aminopolysaccharide derivatives which can be employed in the compositions of this invention. First are the chitosonium polymers. These chitosonium polymers are soluble in water and in mixtures of water and alcohol, readily form humectant films, and are substantive to skin and mucosa. Chitosonium polymer prepared by any method may be utilized in the subject invention. For example, these chitosonium polymers may be prepared by a number of known methods, including direct dissolution, spray drying, lyophilization, and the acid decrystallization process described in International Application Number PCT/US87/01246, published December 17, 1987 as WO 87/07618.

Examples of chitosonium derivatives include those wherein one or more of the amino groups have been neutralized by acids, which may include: pyrrolidone carboxylic, acetic, lactic, glycolic, glyceric, mandelic, salicylic, benzoic, itaconic, malic, nicotinic, glutamic, aspartic, and the acid form of other amino acids such as N-acetyl methionine, N-acetyl tyrosine, N-acetyl glycine, N-benzoyl serine, and the like.

The second type of chitosan derivative included in this invention is covalent derivatives. These derivatives are prepared by the reaction of chitosan with one or more electrophilic reagents such as ethylene oxide, propylene oxide, glycidol, alkyl halides (from $C_1$ to $C_{24}$), glycidyl trialkylammonium salts (alkyl groups from $C_1$ to $C_{24}$), 3-chloro-2-hydroxypropyl ammonium salts, 1,3-propanesultone, haloacetates, succinic anhydride, maleic anhydride, carboxylic acyl halides, the N-carboxy-alpha-amino acid anhydrides, and the like. These chitosan derivatives are readily soluble in water, alcohol, water/alcohol mixtures, or, depending upon the structure of the deivative, may be soluble in ether, acetone, or ethyl acetate. These derivatives are good film formers, good humectants, and are substantive if cationic and/or hydrophobic groups are included in the polymer backbone.

Aminopolysaccharides suitable for use in the subject invention can be conveniently prepared by a method which comprises the steps of:

(a) forming a mixture of a pulverulent, partially deacetylated aminopolysaccharide and

(1) a diluent medium in which the aminopolysaccharide is swellable but essentially insoluble; the medium comprised of:

(i) an inert, water soluble, polar organic diluent in which the aminopolysaccharide is insoluble and the aminopolysaccharide derivative is insoluble; and

(ii) at least one organic acid which is at least partially soluble in water, is sufficiently acidic to form the ammonium salt of the aminopolysaccharide and yet not sufficiently acidic to cause degradation of the aminopolysaccharide or derivative, and which is present in an amount sufficient to protonate the reactive sites of the deacetylated aminopolysaccharide; and

(2) water in an amount up to about 65 weight percent of said medium;

(b) agitating the mixture at a temperature and for a period of time to effect at least partial decrystallization of the aminopolysaccharide to form an aminopolysaccaride derivative; and

(c) recovering the aminopolysaccharide derivative from the mixture.

As described above, a variety of derivatives of decrystallized aminopolysaccharides, such as chitosan, can be prepared. These derivatives can be ionic compositions (salts) or covalent compositions.

To prepare covalent chitosan derivatives such as esters, amides, and ethers, the swollen, decrystallized slurry of the chitosan salt prepared by the aforementioned method, is causticized with a stoichiometric excess of a base such as sodium hydroxide and then reacted with an electrophile, such as ethylene oxide, propylene oxide, glycidol, 1,2-epoxy dodecane, chloroacetic acid, succinic anhydride, and the like.

To prepare ionic derivatives in the form of salts of chitosan, the acid used in the decrystallization step is chosen to provide the desired functional group and both decrystallization and derivatization, i.e. salt formation, is accomplished simultaneously. Alternatively, the organic acid utilized in the decrystallization step can be selected so that the chitosan is not only decrystallized but the salt is obtained containing the desired organic function present in the acid employed.

The decrystallization method described herein differs from other known methods in several respects. First, the acid decrystallization process does not involve dissolution of aminopolysaccharide, such as chitosan, in an aqueous medium. Since chitosan is a very rigid molecule, only a small quantity of chitosan having a moderately high molecular weight (between about 20,000 and greater than 1 million) can be rendered water soluble before the solution becomes too viscous to be easily handled. If the solution is

further diluted to reduce the viscosity, the concentration of chitosan is reduced even further. The dilute nature of such a solution makes chemical reaction to derivatize the molecule inefficient and economically unattractive.

For example, literature currently available from a company engaged in the commercial sale of chitosan in the United States indicates that chitosan is soluble in solutions of most acids, particularly organic acids such as formic acid, malic, tartaric, citric, adipic, and the like. It is further indicated that in order to make a one percent solution of chitosan in water, chitosan is mixed with water and then an equal volume of acid solution is added. For concentrated solutions of chitosan, which are indicated in the literature reference to be from about 2 to 4 percent by weight, an equal weight of acid to that of the chitosan is employed. With inorganic acids such as hydrochloric or nitric acids chitosan is soluble within the range of 0.15 to 1.1 percent acid by weight. Chitosan is not soluble in sulfuric acid and has only marginal solubility in phosphoric acid at concentrations below 0.5 percent.

Thus, the decrystallization method described herein provides the only method known to the inventors whereby aminopolysaccharides are economically decrystallized and derivatized, and recovered, by a simple and efficient process.

A variety of acids can be used in the decrystallization process. It is, of course, necessary that the acid be at least partially soluble in water or hydrophilic media, be sufficiently acidic to form the ammonium salt of the aminopolysaccharide and yet not sufficiently acidic to cause hydrolysis of the aminopolysaccharide or derivative, and be present in an amount sufficient to protonate the reactive sites of the deacetylated aminopolysaccharides.

Such acids can be represented by the formula:

$R\text{-}(COOH)_n$

wherein n has a value of 1 or 2 and R represents a monovalent or divalent organic radical composed of carbon, hydrogen, and optionally at least one moiety selected from the group consisting of oxygen, nitrogen, and sulfur. Combinations of such acids also may be utilized. Preferred acids are the mono- and di-carboxylic acids composed of carbon, hydrogen, oxygen, and nitrogen, which are at least partially water soluble and biologically and/or pharmaceutically acceptable for use in the delivery systems of the present invention.

Accordingly, a wide variety of acids can be employed to simultaneously decrystallize chitosan and provide derivatives suitably utilized in the present invention. Such acids, in addition to those previously identified, include formic, acetic, N-acetylglycine, acetylsalicylic, fumaric, gallic, glycolic, iminodiacetic, itaconic, DL-lactic, maleic, DL-malic, methacrylic, 2-pyrrolidone-5-carboxylic, salicylic, succinamic, succinic, ascorbic, aspartic, adipic, glutamic, glutaric, malonic, nicotinic, pyruvic, sulfonyldiacetic, thiodiacetic, and thioglycolic acids.

The medium employed in the decrystallization of the chitosan is a diluent system combining water and an organic compound. Organic compounds which are useful in this diluent system are those which are water soluble, in which the aminopolysaccharide is insoluble, and in which the aminopolysaccharide derivative is insoluble. Organic compounds suitably employed include acetone, methanol, ethanol, n-propanol, isopropanol, tertiary butyl alcohol, acetonitrile, tetrahydrofuran, dioxane, 2-ethoxyethanol, dimethoxyethane, and the like.

The second component of the diluent medium is water, which is present in an amount up to about 65 weight percent of the total medium, i.e., the total of the water plus the organic compound. In practice, optimum results are obtained when the diluent medium contains from about 30 to about 45 weight percent water and more preferably about 40 weight percent.

In contrast to other methods, the decrystallization described herein avoids formation of a chitosan solution. Rather chitosan is caused to swell and it is unnecessary to form viscous solutions which contain only a few percent chitosan.

The sequence of mixing the diluent medium and the deacetylated chitosan is not critical. However, it has been observed that excellent results are obtained if the diluent medium is prepared from the water and organic compound together with the acid and then the chitosan added.

Chitosan, which is a deacetylated form of chitin, has a very rigid structure, as illustrated in the following formula:

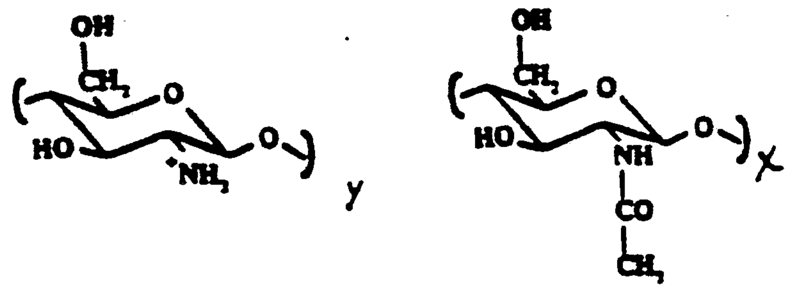

(I)

wherein x is between 0 and 0.5; y is between 0.5 and 1; and x + y = 1. The degree of deacetylation and the molecular weight which occurs in natural chitin depends upon the species, e.g., shellfish from which the chitin is obtained and the method by which it is purified. In chitosan, typical ranges are y is between about 0.5 and 0.9 and x is between about 0.1 and 0.5. Dissolution of chitosan having a low degree of deacetylation in acid solution yields a very viscous product, with a significant insoluble fragment.

For chitosan salts or derivatives to be soluble in water, they should be prepared from starting materials which have a relatively large number of free primary amine groups, i.e., y should be larger than about 0.5. Preferably, the degree of deacetylation of chitosan used herein is in excess of 60 percent, and more preferably in excess of 70 percent. The molecular weight range of chitosan employed in the present invention can range from about 5000 to over a million and more preferably from about 10,000 to about 500,000. Particularly preferred is chitosan having a molecular weight of from about 20,000 to about 250,000.

The above-described mono- and di-carboxylic acids or other combinations can be employed in the preparation of derivatives of chitosan useful in this invention. Thus, derivatives of chitosan suitable for use in the present invention include chitosan salts of carboxylic acids (R′COOH) having the formula:

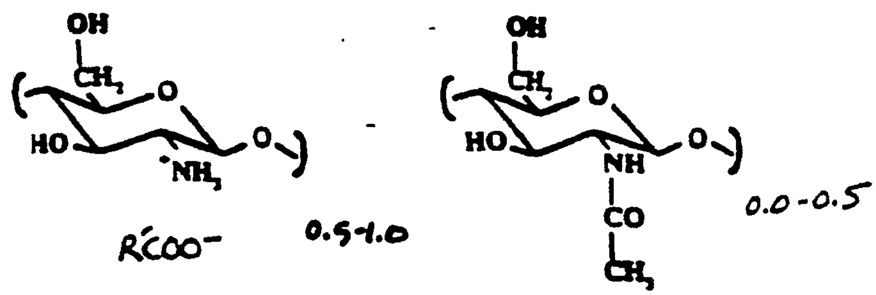

(II)

For example, pyrrolidone carboxylic acid (PCA) is an effective moisturizing agent which has a low order of irritation. Chitosonium pyrrolidone carboxylate has a large number of useful applications such as topical medical formulations. The pyrrolidone carboxylic acid salt of chitosan is useful in delivery systems of the present invention. Such a polymer is prepared by reacting a finely ground slurry of chitosan with PCA in a polar solvent such as aqueous isopropanol or other suitable solvent that will dissolve PCA and swell the chitosan pyrrolidone carboxylic acid salt, but not dissolve it.

Other chitosan derivatives, e.g., salts of other organic acids that are soluble in polar organic solvents such as isopropanol, may be made by this decrystallization process. For example, glycolic acid or lactic acid in aqueous isopropanol can be reacted with chitosan to give the glycolate salt or lactate salt, which also are useful as delivery systems.

When free of its naturally associated proteins, chitin or chitosan is not antigenic to human tissue, and may be used on or inserted under the skin, or placed in contact with body fluids without harm. Chitin in the body is slowly attacked by lysozyme and is absorbed. In addition, chitin and chitosan may be safely ingested by humans, for example, common foods such as bread, beer, wine, shrimp, crabs, and mushrooms all contain some chitin.

In addition to the chiosonium polymers and covalent chitosan derivatives prepared as described above, the delivery systems of the present invention can be comprised of chitosan derivatives prepared by known methods.

Chitosonium polymers suitable for use in the present invention include salts of chitosan prepared with

the following acids:
Acetic
N-Acetyl-L-cysteine
N-Acetyl glycine
Acetylsalicylic
Adipic
L-Aspartic
Citric
Fumaric
2-Furoic
Gallic
L-Glutamic
Glutaric
Glycolic
Hydrochloric
4-Hydroxybenzoic
Iminodiacetic
Itaconic
3-Ketoglutaric
DL-Lactic
Maleic
DL-Malic
Malonic
Nicotinic (Niacin)
2,3-Pyridinedicarboxylic
2-Pyrrolidone-5-carboxylic
Pyruvic
Salicyclic
Succinamic
Succinic
Sulfanilic
Sulfonyldiacetic
L-Tartaric
Thioacetic
Thiolactic
Vanillic
Combinations of these acids also are suitable.

These salts are readily soluble in water at room temperature except for the malate, maleate, itaconate, salicylate, fumarate, and succinate salts which require heating to about 75 °C to effect dissolution, after which they remain soluble. The products from the reaction of acrylic, citric, gallic, 4-hydroxybenzoic and vanillic acids, when used alone, are only slightly soluble in water, because the reaction by which the derivative is formed has limited efficiency.

Chitosan derivatives which can be prepared by the above process include, but are not limited to, chitosonium pyrrolidone carboxylate, chitosonium itaconate, chitosonium niacinate, chitosonium salicylate, chitosonium lactate, chitosonium formate, chitosonium acetate, chitosonium gallate, chitosonium glutamate, chitosonium maleate, chitosonium succinamate, chitosonium aspartate, chitosonium glycolate, and combinations thereof. Each is suitable for use in the subject invention.

In general, the amount of chitosan derivative employed in the compositions of this invention will vary depending upon the particular pharmaceutical or therapeutic active being delivered, whether diluent is present, the type of additives, and the like. In practice, however, it has been found that a concentration of the chitosan derivative in the composition can range up to about 20, preferably between about 0.05 and 10, weight percent, based on the total weight of the composition.

The delivery systems of the invention comprise a non-aqueous fraction which is not soluble in the aqueous fraction. Thus, delivery systems of the invention are emulsions. The non-aqueous, water-insoluble phase can be an active agent, e.g., mineral oil. This phase also may contain an active agent or a diluent.

The delivery systems of the present invention contain pharmaceutical and therapeutic actives that can be applied topically either singularly or in combination. Examples of these actives include, but are not limited to compounds such as the following:

Anti-inflammatory analgesics such as salicylic acid, salicylate esters and salts, acetylsalicylic acid, diflunisal, acetaminophen, phenylbutazone, oxyphenbutazone, sulfinpyrazone, indomethacin, sulindac, fenoprofen, flurbiprofen, ibuprofen, ketoprofen, naproxen, mefenamic acid, floctafenine, tolmetin, zomepirac, diclofenac, piroxicam, and the like.

Local anaesthetics such as cocaine, benzocaine, tetracaine, lidocaine, bupivacaine, their hydrochloride salts, and the like.

Antibiotic agents such as penicillins, cephalosporins, vancomycin, bacitracin, cycloserine, polymyxins, collstin, nystatin, amphotericin B, mupirocim, tetracyclines, chloramphenicol, erythromycin, neomycin, streptomycin, kanamycin, gentamicin, tobramycin, amikacin, netilmicin, spectinomycin, clindamycin, rifampin, nalidixic acid, flucytosine, griseofulvin, and the like.

Sulfanilamide antibacterial agents such as suifanilamide, sulfacetamide, sulfadiazine, sulfisoxazole, sulfamethoxazole, trimethoprim, pyrimethamine, and the like.

Antiviral agents such as vidarabine, acyclovir, ribavirin, amantadine hydrochloride, rimantadine, idoxyuridine, interferons, and the like.

Antiseptic agents such as acridine dyes, alcohols, bronopol, chlorhexidine, phenols, hexachlorophene, organic mercurials, organic peroxides, i.e., benzoyl peroxide, quaternary ammonium compounds, and the like.

Vitamin and vitamin derivatives such as Vitamin A, retinol, retinoic acid (both cis and trans), alpha-tocopherol (Vitamin E), 7-dehydrocholesterol (Vitamin D), Vitamin K, thiamine, riboflavin, niacin, pyridoxine, biotin, pantothenic acid, ascorbic acid, choline, inositol, and the like.

Anti-inflammatory corticosteroids such as progesterone, hydrocortisone, prednisone, fluorocortisone, triamcinolone, dexamethasone, betamethasone, fluocinolone, and the like.

Anti-fungal agents such as miconazole, tolnaftate, naftifine hydrochloride, loceryl, undecylic acid and its salts, and other heterocyclic compounds including morpholine, imidazoles and derivatives thereof.

Vasolidators such as niacin, nicotinate esters and salts, nitroglycerine, amyl nitrite, prazosin, minoxidil and diazoxide; and calcium channel blockers such as nifedipine, diltiazem, indomethacin, and the like.

Gonadal hormones such as gonadotropin-releasing hormone, human chorionic gonadotropin, gonadotropins, 17-beta-estriol, ethinyl estradiol, diethyl stibestrol, norethindrone, norethynodrel, medroxyprogesterone acetate, d-norgestrel, testosterone, fluoxymesterone, androstenedione, norethandrolone, nandrolone phenpropionate, methylandrostenediol, and the like.

Anti-histamines such as diphenhydramine, chlorpheniramine, chlorcyclizine, promethazine, cimetidine, ranitidine, and the like.

Autacoids such as prostaglandins, prostacyclin, thromboxanes, leukotrienes, angiotensins (captopril), as well as other pharmaceutically active peptides such as serotonin, endorphins, vasopressin, oxytocin, and the like.

Kerolytic agents such as benzoyl peroxide, salicylic acid, trichloroacetic acid, and piroctone, and wart treatment compounds such as salicyclic acid, trichloroacetic acid and lactic acid, singularly or in combination with anti-viral agents.

Anti-diarrhea agents such as bismuth salts (especially the subsalicylate), opium and its derivatives, diphenoxylate, difenoxin, loperamide, nufenoxole, lidamine and the like.

Anti-alopecia agents such as niacin, nicotinate esters and salts, and minoxidil.

Steroids such as amcinonide, betamethasone dipropionate, betamethasone valerate, clobestasol propionate, desonide, desoximetasone, diflorasone diacetate, fluocinolone, flucinolone acetonide, fluocinonide, halcinonide, hydrocortisone, hydrocortisone valerate, mometasone furoate, triamcinolone, triamcinolone acetonide, triamcinolone acetonide USP, and tridesilon desonide.

Moisturizing agents such as lactic acid, pyrrolidone carboxylic acid, glycolic acid, glycerine, propylene glycol, sorbitol, other alphahydroxy carboxylic acids, and various salts of these esters and salts, and the like.

As indicated above, this list of pharmaceutical and therapeutic actives is not inclusive, but is presented merely to demonstrate the scope of the invention. A wide variety of other actives, such as permethrin for veterinary applications, can be employed, either alone or in combination. Similarly, omentum may be delivered by a delivery system of the invention.

Omentum products, also known as omental lipid materials, are found to be useful as cosmetics for skin creams and such for topical use as skin softeners having an emollient, softening, and smoothing effect. Omentum is safe to use in the presence of cuts and abrasions and in skin conditions such a kertosis and irritations.

Mammalian omental extracts are said to be useful in the sof tening, moisturizing, and smoothing of skin, and reduction of calluses and white spots thereon, and, therefore, useful as a skin cream or lotion. It is also

said to be useful in accelerating the rate of tanning, thus reducing the time required in the sun to achieve the tanning effect. Since such sun exposure is well known to lead to skin conditions such as melanoma or other skin cancers, omentum can be said to be useful to prevent such conditions.

Omental materials also are said to be used in wound healing, as described above, in angiogenesis for myocardial conditions such as myocardial infarcts, angina, vascular or coronary inplants, angioplasty, and bone healing.

Glycosaminoglycans (GAGS) comprise a class of polysaccharides that occur in the connective tissue of mammals, and include hyaluronic acid, chondroitin sulfate, and heparin, and which can be included in delivery systems of the invention. Some of these polysaccharides, hyaluronic acid in particular, have been used successfully for wound healing and tissue regeneration in both humans and laboratory animals. The exact mechanism of tissue regeneration is not known, but oligomeric metabolites of N-acetylglucosamines and glucosamine functionality present in glycosaminoglycans such as hyaluronic acid is present in chitin and chitosan, and similar wound healing and tissue regeneration properties have been reported for chitin and chitosan.

Preferably, hyaluronic acid is added only to delivery systems containing covalent derivatives of chitosan. Hyaluronic acid interacts with chitosonium salts and may cause precipation thereof.

The amount of active employed will be that amount necessary to deliver a pharmaceutically or therapeutically effective amount to achieve the desired result at the site of application. In particular, an effective amount depends, inter alia , upon the particular active, the severity of the condition, and other factors. In general, the concentration of the actives in the delivery systems can vary from as little as 0.001 up to 50 percent or higher, by weight of the delivery system. More typically, the concentration of a pharmaceutical active is between about 0.01 and 10 wt percent of the delivery system, while that of a therapeutic active typically is between about 0.1 and 50 percent. Skilled practitioners will be able to adjust the quantity of active in the delivery system.

If desired, the delivery systems of this invention can contain one or more pharmaceutically acceptable diluents or vehicles in addition to the chitosan derivative, the active component, and the non-aqueous water-insoluble fraction. In many instances, the chitosan derivative itself can be about 0.5 to about 20 weight percent of the system with the remainder being diluent and optionally, other additives. Suitable diluents include among others, water, ethanol, aqueous ethanol, isopropanol, glycerine, dimethylether, polyethylene glycol, ethoxylated or propoxylated glucose, sorbitol derivatives, and the like.

Additives for the enhanced percutaneous absorption of various pharmaceutical or therapeutic actives also may be utilized. Such percutaneous enhancers include propylene glycol, glycerol, urea, diethyl sebecate, sorbitan ethoxylates, nicotinate esters (such as hexyl nicotinate), oleic acid, pyrrolidone carboxylate esters, (such as dodecyl pyrrolidone carboxylate), N-methyl pyrrolidone, N,N-diethyl-m-toluamide, dimethyl sulfoxide, decyl methyl sulfoxide, alkyl methyl sulfoxides, N,N-dimethyl formamide, cis -11-octadecenoic acid, 1-dodecylazacycloheptan-2-one, and 1,3-dioxacyclopentane or 1,2-dioxacyclohexane containing at least one aliphatic group of four to eighteen carbon atoms.

Incorporation of delivery enhancers into delivery systems of the present invention improves absorption of the active at the application site. Delivery enhancers are useful whenever the active is to be delivered through the stratum corneum. The improved efficiency of delivery can be utilized in a number of ways. For example, it affords the opportunity to deliver, in a topical system, an unexpectedly high concentration of active. A high concentration of active could be utilized to reduce the number of times the treatment must be applied during a given period. Skilled practitioners recognize other manners of utilizing the unexpected results obtained herein.

Delivery enhancers preferably are not completely hydrophobic or oleophobic. Rather, they are both hydrophilic and oleophilic to varying degrees. Thus, delivery enhancers may be at least slightly soluble in aqueous solutions and in a non-aqueous component.

Some delivery enhancers also can act as an emulsifier between the water-insoluble, non-aqueous fraction and the aqueous fraction. Addition of such an emulsifying delivery enhancer before the emulsion is formed typically causes the drop size of the oil phase to be smaller. Smaller drop size may contribute to increased delivery efficiency. Benzyl alcohol, dimethyl sulfoxide, and AZONE are suitable.

Delivery enhancers are distinct from the above-described percutaneous enhancers. The percutaneous enhancers typically act as humectants, lubricants, softening agents, moisturizers, debris removers, and impart cleansing and other effects. These enhancers therefore prepare the application site to receive active by ensuring that the site is softened, free of debris, and amenable to penetration. In contrast, delivery enhancers do not serve such functions. Rather, delivery enhancers serve to provide a path or bridge through the skin, reduce the hydrophobicity of the skin, or otherwise deliver the active more efficiently.

Certain compositions serve to further enhance the effect of the delivery enchancers. For example,

propylene glycol, described above as a percutaneous enhancer, further improves the delivery of steroids when benzyl alcohol also is utilized as a delivery enhancer. Propylene glycol thus is a preferred percutaneous enhancer in conjunction with benzyl alcohol as a delivery enhancer. Hexylene glycol and cis - octadecen-11-oic acid also are compositions which enhance delivery.

As defined herein, mineral oil is a therapeutic active, because it serves as an excellent emollient. However, the oil typically imparts an unpleasant "oily" feeling to the skin if applied directly in a known delivery system. However, if applied as part of a delivery system of the invention, the oily feeling on the skin is not present.

Delivery systems of the invention also are suitable for veterinary uses, as described above. For example, permethrin insecticide is delivered together with an emollient by a system of the invention.

Another example of the unexpected results obtained by using a delivery system of the invention includes delivery of omentum products. Because additional carriers, such as a blend of the benzoates of $C_{12}$-$C_{15}$ alcohols (Fine Solv TN), can be added to the delivery system, omentum can be delivered more conveniently at concentrations higher than those achieved by known methods.

The delivery systems of the present invention are particularly efficient. The chitosan salt forms a substantive film over the application site, ensuring that the active does not migrate and is not removed from the site, e.g., by perspiration or contact with clothing. The excellent site protection provided by the chitosan film affords the opportunity to deliver to a site an increased quantity of active per application. Therefore, instead of requiring, e.g., three or more applications daily, as may be utilized with known delivery systems, the delivery system of the present invention may be applied only once daily. This decreased application frequency provides several benefits, including increased consumer acceptance and improved treatment derived, inter alia , from an easily understood and executed treatment schedule.

Known methods for topically delivering actives typically require repeated applications during a 24-hour period to ensure that a sufficient quantity of active is delivered to the site. Repeated application is at best inconvenient, and at worst leads to uneven treatment, such as lack of treatment because the scheduled application time fell during a period of sleep, or at a time when application was impossible.

Repeated application of known topical treatments is necessary because the treatments cannot deliver a sufficient quantity of active in one application, e.g., without feeling greasy or delivering the active at an uneven rate. However, the delivery system of the invention affords an even delivery rate over a long period.

Delivery systems of the invention comprise oil-in-water emulsions. Oil-in-water emulsions feel relatively "non-greasy" when applied, whereas water-in-oil emulsions tend to have a greasy or oily feel. Therefore, oil-in-water emulsions are preferred by consumers.

Emulsion-type delivery systems of the invention are made by the "direct" method or by the "inversion" method. In the "direct" method, the oil phase is dispersed into the continuous aqueous phase to form the oil-in-water emulsion directly. An oil-in-water emulsion is made by the "inversion" method by emulsifying the aqueous phase into a continuous oil phase. At first, a water-in-oil emulsion is formed, but, as the quantity of aqueous phase is increased, the emulsion becomes "inverted" and forms an oil-in-water emulsion. Either preparation method can be used to prepare emulsion-type delivery systems of the invention.

An active may be part of either fraction of the delivery system. The active may be added to the system either as part of one of the fractions, or a separate component after the remainder of the delivery system is formed. In the latter technique, the active is distributed by agitation.

in practice, the delivery systems of the invention are readily formulated by mixing a non-aqueous fraction with a solution or suspension of the chitosan derivative. The active or actives are dissolved or suspended in either fraction, in a component of a fraction, or comprise the fraction, as in the case of an emollient where the active is mineral oil, a non-aqueous water-insoluble composition. For example, an active may be incorporated into the aqueous fraction by first dissolving it in a water-soluble non-aqueous component, such as propylene glycol or isopropanol. The non-aqueous fraction is mixed with a solution or suspension of the desired chitosan derivative. An emulsion is formed if another non-aqueous fraction is not soluble in the aqueous phase; a suitable emulsifier may be used. Other adjuvant ingredients such as glycerine, propylene glycol, sorbitol, preservatives, stearic acid, cetyl alcohol, other high molecular weight alcohols, surfactants, menthol, eucalyptus oil, other essential oils, fragrances, penetration enhancers, and the like to give stable delivery systems, such as cremes, ointments, lotions, and aerosols, may also be included.

The substantivity of delivery systems of the invention and other systems is illustrated by ESCA (Electron Spectroscopy for Chemical Analysis) testing on an artificial skin substitute to which a delivery system of the invention is applied. Information gathered from these tests compares favorably with ESCA testing of the substantivity of chitosonium lactate solutions with human skin, as described herein.

ESCA is a known technique for qualitative and quantitative surface analysis for simple compositions of matter, such as whether silicone is present on a substrate. Electronspectrochemical analysis has been utilized to determine whether hair conditioner builds up on the surface of hair. However, the substantlvity of many topical delivery systems cannot be directly determined in this traditional, exterior surface-measuring technique, because the thickness of the film formed on the skin by delivery systems of the invention may exceed 100 Angstroms. Typically, a maximum depth of about 100 Angstroms can be analyzed by the technique.

It has been discovered that the substantivity of topical preparations, including films formed by delivery systems of the invention, can be measured by applying the delivery system to a substrate, applying tape to the treated area, peeling off the tape, and analyzing both the material which remains on the substrate (where possible) and the material which is transferred to the tape. Skilled practitioners recognize that when testing substantivity on hydrophobic or oleophobic. Rather, they are both hydrophilic and oleophilic to varying degrees. Thus, delivery enhancers may be at least slightly soluble in aqueous solutions and in a non-aqueous component.

Some delivery enhancers also can act as an emulsifier between the water-insoluble, non-aqueous fraction and the aqueous fraction. Addition of such an emulsifying delivery enhancer before the emulsion is formed typically causes the drop size of the oil phase to be smaller. Smaller drop size may contribute to increased delivery efficiency. Benzyl alcohol, dimethyl sulfoxide, and AZONE are suitable.

Delivery enhancers are distinct from the above-described percutaneous enhancers. The percutaneous enhancers typically act as humectants, lubricants, softening agents, moisturizers, debris removers, and impart cleansing and other effects. These enhancers therefore prepare the application site to receive active by ensuring that the site is softened, free of debris, and amenable to penetration. In contrast, delivery enhancers do not serve such functions. Rather, delivery enhancers serve to provide a path or bridge through the skin, reduce the hydrophobicity of the skin, or otherwise deliver the active more efficiently.

Certain compositions serve to further enhance the effect of the delivery enchancers. For example, propylene glycol, described above as a percutaneous enhancer, further improves the delivery of steroids when benzyl alcohol also is utilized as a delivery enhancer. Propylene glycol thus is a preferred percutaneous enhancer in conjunction with benzyl alcohol as a delivery enhancer. Hexylene glycol and cis - octadecen-11-oic acid also are compositions which enhance delivery.

As defined herein, mineral oil is a therapeutic active, because it serves as an excellent emollient. However, the oil typically imparts an unpleasant "oily" feeling to the skin if applied directly in a known delivery system. However, if applied as part of a delivery system of the invention, the oily feeling on the skin is not present.

Delivery systems of the invention also are suitable for veterinary uses, as described above. For example, permethrin insecticide is delivered together with an emollient by a system of the invention.

Another example of the unexpected results obtained by using a delivery system of the invention includes delivery of omentum products. Because additional carriers, such as a blend of the benzoates of $C_{12}$-$C_{15}$ alcohols (Fine Solv TN), can be added to the delivery system, omentum can be delivered more conveniently at concentrations higher than those achieved by known methods.

The delivery systems of the present invention are particularly efficient. The chitosan salt forms a substantive film over the application site, ensuring that the active does not migrate and is not removed from the site, e.g., by perspiration or contact with clothing. The excellent site protection provided by the chitosan film affords the opportunity to deliver to a site an increased quantity of active per application. Therefore, instead of requiring, e.g., three or more applications daily, as may be utilized with known delivery systems, the delivery system of the present invention may be applied only once daily. This decreased application frequency provides several benefits, including increased consumer acceptance and improved treatment derived, inter alia , from an easily understood and executed treatment schedule.

Known methods for topically delivering actives typically require repeated applications during a 24-hour period to ensure that a sufficient quantity of active is delivered to the site. Repeated application is at best inconvenient, and at worst leads to uneven treatment, such as lack of treatment because the scheduled application time fell during a period of sleep, or at a time when application was impossible.

Repeated application of known topical treatments is necessary because the treatments cannot deliver a sufficient quantity of active in one application, e.g., without feeling greasy or delivering the active at an uneven rate. However, the delivery system of the invention affords an even delivery rate over a long period.

Delivery systems of the invention comprise oil-in-water emulsions. Oil-in-water emulsions feel relatively "non-greasy" when applied, whereas water-in-oil emulsions tend to have a greasy or oily feel. Therefore, oil-in-water emulsions are preferred by consumers.

Emon is 5 cP), 12 parts purified water, and 5.6 parts Tween 80 (surfactant from ICI Americas, Inc.;

polyoxyethylene (20) sorbitan mono-oleate).

The non-aqueous phase of the comparative composition was 50 parts Omentum (a proprietary formulation of Angio Medical Corp., Van Vuys, CA), and 2.4 parts Span 80 (polyoxyethylene (20) sorbitan mono-oleate from ICI Americas, Inc.).

The non-aqueous phase of the delivery system of the invention was 50 parts Omentum, 2.4 parts Span 80, and 5 parts Fine Solv TN (benzoate of $C_{12}$-$C_{15}$ alcohols; Finetex, Inc., Elmwood Park, NJ).

Each emulsion was formed by heating each phase to 75 °C, then directly forming the emulsion by adding the non-aqueous phase to the aqueous phase with good mixing.

Each emulsion was cooled to 50 °C and 1 part benzyl alcohol was added. Equal portions of the delivery system without the carrier and the delivery system with the carrier were applied to a human hand. After 3 minutes, the delivery system without the carrier felt greasy and imparted a greasy look to the skin. Without Fine Solv TN or an equivalent carrier, at 50 percent Omentum, the system was cosmetically unpleasant. However, after the same period, the site to which the delivery system which incorporated a carrier was applied was non-greasy and both tactilely and visually pleasant.

## Example 3

A permethrin insecticide intended for veterinary use was made in accordance with the method of the invention.

One hundred parts of analytical grade isopropyl alcohol containing 5.25 parts of permethrin were added slowly to 369.75 parts of a 3 percent solution of chitosan pyrrolidone carboxylate at a temperature of 50 °C. Slow addition, i.e., over a 30-minute period, was necessary to avoid precipitation of the chitosan pyrrolidone carboxylate. Then, the temperature was raised to 72 °C.

A mixture of 22.5 parts Promulgen D (cetearyl alcohol, a blend of cetyl alcohol and stearyl alcohol, and ceteareth 20) and 2.5 parts glycerol monostearate at 80 °C was emulsified into the chitosan-containing solution. The temperature was reduced to 50 °C, at which time 2.5 parts of benzyl alcohol were added. Stirring was continued until the temperature was about 40 °C.

This delivery system of the invention was effective as an emollient and permethrin delivery system.

## Example 4

To test the substantivity of topically-applied substances, including delivery systems of the invention, a small quantity of substance is rubbed onto a carboxyl group-containing polyvinylchloride polymer film, much as it would be applied to the skin by a consumer. The treated substrates were examined both directly after application of the formulation and after washing the treated area with a 1 percent solution of sodium lauryl sulfate for three minutes, followed by a ten-minute rinse in running tap water. Several commercially available emollients were also examined under identical sample preparation conditions.

ESCA analyses were performed using a Surface Science Instruments SSX-100 ESCA spectrometer. Analyses were performed on each sample using a 1 mm diameter analysis area. In most cases, two analyses from different areas of the samples were performed. All measured binding energies were charge-corrected to the principal C1s photolectron line at 284.6eV. Angular-resolved measurements were performed using a tilt stage accessory supplied by Surface Science Instruments.

### A. Comparison of Delivery System of the Invention to Commercial Emollient

A composition of the invention (emollient) comprising chitosonium lactate and silicone was compared to a fatty acid- and silicone-containing commercial emollient (COMPLEX 15). The ESCA data in Table 1 illustrate the presence of the silicone ingredient in both preparations.

The data in Table 1 illustrate that, for the commercial emollient, no chlorine from the substrate was detected immediately after application, indicating that the thickness of the emollient exceeded the 50-100 Angstrom sampling depth of the ESCA technique. The as-applied system of the invention allowed detection of a small amount of chloride. However, an angular-resolved ESCA experiment on this sample (Table 2) showed a smooth decline in the amount of chlorine detected as the sampling depth was made shallower.

This data indicated that the chlorine observed in the as-applied sample of the invention was the result of a continuous layer of the delivery system which was thinner than the sampling depth, rather than a discontinuous coating. A discontinuous coating, in which bare patches of substrate were present, would not be expected to show the smooth decline in chloride concentration.

TABLE 1

SURFACE COMPOSITION: VINYL SUBSTRATES

| Sample | C | Cl | O (Atom. %) | N | Si | S | Other |
|---|---|---|---|---|---|---|---|
| Control | 69.7 | 19.3 | 8.4 | 0.8 | 1.0 | -- | 0.9 Na |
| | 70.1 | 17.4 | 9.4 | 0.7 | 1.3 | -- | 1.1 Na |
| Control Washed | 73.6 | 13.2 | 10.9 | 1.0 | 1.2 | -- | -- |
| | 72.6 | 13.3 | 11.6 | 1.2 | 1.2 | -- | -- |
| COMPLEX 15 | 56.5 | -- | 23.0 | -- | 20.5 | -- | -- |
| | 60.1 | -- | 20.7 | -- | 19.1 | -- | -- |
| COMPLEX 15/ Washed | 74.0 | 14.1 | 11.0 | -- | 0.4 | 0.3 | -- |
| | 74.1 | 14.7 | 9.8 | -- | 1.4 | -- | -- |
| Invention | 59.4 | 3.5 | 20.3 | -- | 16.9 | -- | -- |
| | 59.0 | 5.3 | 20.1 | -- | 15.6 | -- | -- |
| Invention Washed | 69.2 | 15.0 | 14.0 | 1.2 | 0.4 | 0.2 | -- |
| | 65.3 | 7.6 | 21.6 | 2.9 | 1.6 | 0.5 | Na,Ca |

TABLE 2

ANGULAR RESOLVED ESCA OF AS-TREATED SAMPLE OF INVENTION

| Angle* | C | Cl | O | Si | Other |
|---|---|---|---|---|---|
| 80 | 62.4 | 7.6 | 18.4 | 11.3 | 0.3% N |
| 35 | 59.9 | 4.1 | 19.9 | 16.1 | -- |
| 20 | 57.3 | 2.9 | 22.1 | 17.7 | -- |

*Surface sensitivity increases as this angle decreases.

The washed samples show radical differences from the as-applied samples in both instances. COMPLEX 15 appeared to be almost completely removed by the wash procedure. The surface chlorine level rose from nondetectable to slightly above that detected on the untreated control vinyl following the wash step. There were indications in the carbon high resolution data of possibly some slight retention of a fatty acid/ester type material. However, based on the ESCA data, there was no evidence of substantial retention of any component of this product by this vinyl substrate.

The situation was demonstrably different in the case of the delivery system of the invention. While the

silicone component was again readily removed by the surfactant wash, the chitosonium salt remained substantive. This conclusion was based on the detection of the marker functional groups, quaternary ammonium and C-O, on the washed substrates. As shown in Table 1, relatively high levels of nitrogen were detected on this sample. More importantly, nitrogen high resolution spectra clearly showed that the additional nitrogen observed on the washed treated sample was in the quaternary form. The nitrogen observed in the high resolution spectra did not show the expected ratio of quaternary to amide type nitrogen for the chitosonium polymer. The quaternary form should be the predominant nitrogen species if all the nitrogen is due to chitosonium. The data indicate that an additional nitrogen species is present, possibly due to contamination in the wash procedure. This possibility is suggested by the data from the washed vinyl control which also showed a nitrogen species with a binding energy similar to that given by the amide. However, it must be emphasized that this effect in no way diminishes the ability to conclusively identify the chitosonium since the quaternary nitrogen series is unique to that molecule.

Further evidence for chitosonium retention was found in the oxygen content, which also tracked with the nitrogen level as would be expected for the chitosonium structure, and a carbon high resolution spectrum, which showed the high level of ether-type (C-O and O-C-O) carbon expected for this molecule. The chlorine level was also inversely related to the nitrogen content, again consistent with increased amounts of chitosonium polymer yielding more complete masking of the substrate. Finally, low levels of sulfur were detected on both areas of this sample, with a higher sulfur level correlating with the higher nitrogen level. It seems reasonable to attribute this to residual sulfate surfactant retained by the cationic chitosonium. Similar retention of sodium dodecylsulfate by cationic cellulosics has been previously documented by ESCA.

Clear evidence for the presence of the polymer was observed in locations examined. This was particularly noteworthy in comparison to COMPLEX 15, for which a thicker film of the emollient was initially deposited on the vinyl and yet was readily removed. A very thin film (less than 100 Angstrom) of the delivery system of the invention was initially deposited, yet retention of at least the chitosonium component of the formulation was achieved, even after the relatively strenuous washing and rinsing conditions employed.

B. Comparison of Delivery System of the Invention to Commercial Emollients

A second siloxane- and chitosonium lactate-containing delivery system (emollient) of the invention was compared to commercial emollients. The data in Table 3 illustrate the results of the test.

TABLE 3

SURFACE COMPOSITION; VINYL SUBSTRATES

| Sample | Atom % Composition C | Cl | O | N | Si | S | Other |
|---|---|---|---|---|---|---|---|
| Control | 74.3 | 17.5 | 8.2 | -- | -- | -- | -- |
| Washed control | 71.5 | 18.3 | 8.7 | 0.6 | 0.9 | -- | -- |
| MOISTUREL | 52.1 | -- | 23.2 | -- | 24.7 | -- | -- |
| Washed MOISTUREL | 82.7 | 9.5 | 6.4 | 0.1 | 1.4 | -- | -- |
| Washed MOISTUREL | 79.2 | 9.2 | 9.3 | -- | 2.4 | -- | -- |
| NUTRADERM | 86.0 | -- | 12.6 | 0.4 | -- | 0.7 | 0.3%Na |
| Washed NUTRADERM | 72.8 | 18.7 | 8.2 | 0.2 | -- | -- | -- |
| LAC.HYDRIN | 83.9 | 0.1 | 14.7 | 1.3 | -- | -- | -- |
| Washed LAC.HYDRIN | 72.0 | 20.2 | 7.8 | -- | -- | -- | -- |
| EUCERIN | 98.6 | -- | 1.4 | -- | -- | -- | -- |
| Washed EUCERIN | 99.1 | 0.4 | 0.3 | -- | 0.2 | -- | -- |
| Invention | 61.2 | 0.3 | 20.7 | -- | 17.8 | -- | -- |
| Washed Invention | 67.7 | 0.3 | 23.4 | 2.2 | 4.1 | 2.3 | -- |
| 10-washed Invention | 69.4 | 10.6 | 16.0 | 1.1 | 2.2 | 0.7 | trace Cu |

The high chlorine levels of the washed NUTRADERM and LAC.HYDRiN samples, which are essentially identical to the washed control chlorine levels, indicate that neither product is substantive. The essential absence of chlorine in the as-applied test and the high chlorine level of the as-washed sample illustrate the initial presence and non-substantive nature of the products. It is worth noting that LAC.HYDRIN does contain a quaternary nitrogen species as a component. This species was readily detected on the as-dried surface. However, in this material, the quaternary nitrogen species is the ammonium anion which serves as a counterion to an anionic lactate group. While this cationic ammonium might possibly be expected to be retained due to ionic interaction with the substrate, it cannot serve to anchor any component of the formulation. Indeed, the ESCA data indicated that the washing conditions used in these experiments were sufficient to remove the ammonium as well.

EUCERIN was relatively unaffected by the wash procedure used. EUCERIN is a water-in-oil ointment which leaves a greasy surface which does not wash off. As can be seen from the data, this particular material is essentially a hydrocarbon ointment which resisted the surfactant washing quite well. MOISTUREL showed some substantivity, as the chlorine content of the washed sample is intermediate those of the as-applied and control samples. As applied, it gave essentially a pure polydimethylsiloxane surface due to the silicone oil in the formulation. The polydimethylsiloxane was almost totally removed by the surfactant wash but some component of the formulation was retained. Therefore, this sample was examined using the angular-resolved technique. The results of this analysis (Table 4) showed enhanced carbon concentration at the immediate surface indicating that the material retained from this formulation was a hydrocarbon oil. Petrolatum is the major component of the formulation and, in all likelihood, this is the material retained, similar to the hydrocarbon retention observed in the EUCERIN sample.

TABLE 4

ANGULAR-RESOLVED ESCA ANALYSIS OF WASHED MOISTUREL SAMPLE

| | Atom % | | | | | |
|---|---|---|---|---|---|---|
| Angle* | C | O | Cl | N | Si | Mg |
| 80 | 73.9 | 10.5 | 10.0 | 0.5 | 3.3 | 1.5 |
| 35 | 82.2 | 7.9 | 7.8 | -- | 2.1 | -- |
| 20 | 85.1 | 6.7 | 5.8 | -- | 2.3 | -- |

*Surface sensitivity increases as this angle decreases.

The chitosonium lactate-containing sample of the invention was clearly substantive. As in Example 4A, the data from the washed sample indicated substantial heterogeneity; however, the chitosan polymer was clearly identified in both areas analyzed. (The washed sample was visibly heterogeneous as well, with areas of the surface covered by a white film and others which appeared very clean. The data in the Table 4 were taken from both regions, with the higher chlorine content observed in the "clean" area of the washed sample.) The carbon and nitrogen high resolution data from a washed sample showed the C-O, O-C-O, and quaternary nitrogen groups, including the expected quatenary-to-amide nitrogen ratio, quite distinctly.

Table 5 contains data on the compositions of the substrate surface and of the tape surface after the substrate was stripped with tape as described in the specification.

TABLE 5

SURFACE COMPOSITIONS FOLLOWING TAPE STRIPPING

| Sample | Atom % C | O | Cl | Si | N | Other |
|---|---|---|---|---|---|---|
| Washed MOISTUREL sample: | | | | | | |
| Vinyl Interface Surface | 75.1 | 10.8 | 11.0 | 3.2 | -- | -- |
| Tape Interface Surface | 85.3 | 14.7 | -- | -- | -- | -- |
| As Dried Invention sample 4B: | | | | | | |
| Vinyl Interface Surface | 67.2 | 20.2 | 0.5 | 11.4 | 0.8 | -- |
| Tape Interface Surface | 59.1 | 23.6 | 0.3 | 17.2 | 0.1 | -- |
| Washed Invention sample 4B: | | | | | | |
| Vinyl Interface Surface | 68.3 | 21.2 | 2.9 | 4.2 | 1.8 | -- |
| Tape Interface Surface | 69.7 | 23.4 | 0.4 | 2.8 | 2.1 | -- |
| Adhesive Tape Surface | 84.2 | 15.8 | -- | -- | -- | -- |

Tape stripping did not remove a significant amount of the deposited MOISTUREL components. As noted above, this material is believed to deposit a hydrocarbon film on the vinyl substrate. Comparison of the relative levels of carbon and oxygen on the tape surface stripped from the MOISTUREL sample and the control data for the tape adhesive surface alone showed a slight transfer of hydrocarbon to the tape. Essentially, therefore, the tape adhesive shows no ability to remove this film. If only the tape surface were available for analysis, as would be the case for an experiment utilizing a human subject, no deposition of the material would be detected.

The sample of the invention showed transfer of the siloxane component to the tape and, as evidenced by the nitrogen detected, transfer of some of the chitosonium polymer as well. The presence of the chitosonium on both interface surfaces was again confirmed by the high resolution data. The combination of the tape stripping results with the data on the as-treated substrate (Table 3) of this sample illustrated that the delivery system of the invention forms a layered structure upon application, with the chitosonium polymer present at or near the vinyl interface and the siloxane polymer forming a film over this at the air interface.

The data from the washed sample of the delivery system of the invention are virtually identical. The only difference is the level of chlorine detected; this level would be expected to be higher on the vinyl substrate surface. The chitosonium polymer is readily detected on both interfaces. The delivery system has such good substantivity that tape stripping essentially produces cohesive failure within the deposited chitosonium film. This is dramatically different from the situation observed on the MOISTUREL sample, and further emphasizes the differences not only degrees of retention, but also that this technique, i.e., the tape stripping method, an effective way to determine substantlvity on living tissues.

Although particular embodiments of the invention have been discussed herein, those skilled in the art will appreciate that changes and modifications may be made without departing from the spirit of the invention, as defined in and limited only by the scope of the appended claims.

## Claims

1. A biocompatible, substantive, film forming delivery system for delivery of pharmaceutical or therapeutic actives to a desired topical site of a subject, said system comprising an emulsion comprising;

A) at least one pharmaceutical or therapeutic active;

B) an aqueous fraction comprising at least one aminopolysaccharide derivative selected from the group consisting of chitosonium polymers and covalent chitosan derivatives: and

C) a non-aqueous fraction which is not soluble in the aqueous fraction,

and wherein said system, after delivery to said site, provides a substantive, gas permeable film from which the actives are available for treatment of said subject at said site.

2. The delivery system of claim 1 wherein said chitosan derivative is a chitosonium polymer.

3. The delivery system of claim 1 wherein said chitosan is a covalent chitosan derivative, e.g. chitosonium pyrrolidone carboxylate, chitosonium niacinate, chitosonium itaconate, chitosonium salicylate, chitosonium lactate or chitosonium glycolate.

4. The delivery system of claim 3 wherein said chitosan derivative is blended with hyaluronic acid.

5. The delivery system of at least one of the claims 1-4 which contains a pharmaceutically acceptable diluent.

6. A method for the preparation of a delivery system for use in the administration of pharmaceutical and therapeutic actives to a topical site of a subject, said delivery system comprising:

A) at least one pharmaceutical or therapeutic active;

B) an aqueous fraction comprising at least one chitosan derivative selected from the group consisting of chitosonium polymers and chitosan derivatives; and

C) a non-aqueous fraction which is not soluble in the aqueous fraction;

which method comprises emulsifying the active, the aqueous fraction, and the non-aqueous fraction to form an emulsion.

7. The method of claim 6 which includes a diluent.

8. The method of at least one of the claims 6-7 wherein said chitosan derivative is dissolved in a solvent before blending.

9. The method of at least one of the claims 6-8 wherein said active component is dissolved in a solvent before blending.

10. A method for determining the substantivity under preselected conditions of a delivery system for a pharmaceutical or therapeutic active to a substrate, said method comprising:

A) exposing a treated area formed by applying delivery system to the substrate to conditions under which the substantivity is to be determined;

B) applying to the exposed treated area means for removing from the substrate at least part of the delivery system;

C) separating the removing means from the exposed treated area; and

D) determining by electronspectrochemical analysis whether the delivery system is present on the removing means.

11. The method of claim 10 wherein the substrate is living tissue.

12. The method of claim 10 wherein the substrate is a carboxyl group-containing polyvinyl chloride polymer having a negative surface potential similar to that of human skin, said method further comprising:

(E) determining by electronspectrochemical analysis whether at least a fraction of the delivery remained on the substrate after the separation of step(c).